(19) Europäisches Patentamt
European Patent Office
Office européen des brevets

(11) Veröffentlichungsnummer: **0 014 675**
**B1**

(12) # EUROPÄISCHE PATENTSCHRIFT

(45) Veröffentlichungstag der Patentschrift: **30.11.83**

(51) Int. Cl.³: **C 07 C 149/437,** A 01 N 47/34

(21) Anmeldenummer: **80810021.8**

(22) Anmeldetag: **28.01.80**

(54) **Substituierte N-Phenyl-N'-benzoylharnstoffe, Verfahren zu ihrer Herstellung, diese Verbindungen enthaltende Mittel und ihre Verwendung zur Bekämpfung von Schädlingen.**

(30) Priorität: **01.02.79 CH 983/79**
**15.10.79 CH 9261/79**

(43) Veröffentlichungstag der Anmeldung:
**20.08.80 Patentblatt 80/17**

(45) Bekanntmachung des Hinweises auf die Patenterteilung:
**30.11.83 Patentblatt 83/48**

(84) Benannte Vertragsstaaten:
**AT BE CH DE FR GB IT NL**

(56) Entgegenhaltungen:
**DE - A - 2 601 780**
**DE - A - 2 726 684**
**US - A - 4 089 975**

**J. Agr. Food Chem. Vol. 21, No. 3, pages 348-354, 1973**

(73) Patentinhaber: **CIBA-GEIGY AG, Patentabteilung Postfach, CH-4002 Basel (CH)**

(72) Erfinder: **Ehrenfreund, Josef, Dr., Langenhagweg 11, CH-4123 Allschwil (CH)**

**EP 0 014 675 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Substituierte N-Phenyl-N′-benzoylharnstoffe, Verfahren zu ihrer Herstellung, diese Verbindungen enthaltende Mittel und ihre Verwendung zur Bekämpfung von Schädlingen

Die vorliegende Erfindung betrifft neue substituierte N-(4-Alkenylthio)-phenyl-N′-benzoylharnstoffe, Verfahren zu ihrer Herstellung und ihre Verwendung in der Schädlingsbekämpfung.

Die erfindungsgemäßen substituierten N-(4-Alkenylthio)-phenyl-N′-benzoylharnstoffe haben die Formel I

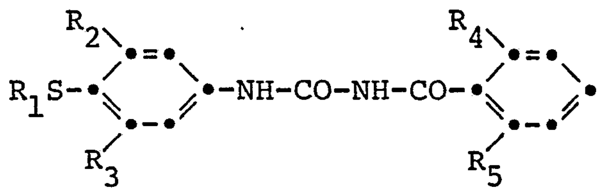

(I)

worin
$R_1$ den Rest $-CH_2-CH=CH-R_6$;
$R_2$ und $R_3$ Chlor;
$R_4$ Wasserstoff oder Fluor;
$R_5$ Fluor; und
$R_6$ Chlor oder Brom
bedeuten.

Die Verbindungen der Formel I fallen im allgemeinen als cis/trans-Isomerengemische an. In diesem Sinne sind als derartige Verbindungen der Formel I sowohl die cis- oder trans-Formen als auch die entsprechenden Isomerengemische zu verstehen. Ein Isomerengemisch kann z. B. mit Hilfe der bekannten chromatographischen Trennmethoden und anschließender Eluierung in die isomeren Formen getrennt werden. Zur Synthese stereochemisch einheitlicher Verbindungen der Formel I verwendet man mit Vorteil stereochemisch einheitliche Ausgangsverbindungen der nachstehenden Formeln II oder IV.

Die Verbindungen der Formel I können nach an sich bekannten Verfahren hergestellt werden (vgl. u. a. die deutschen Offenlegungsschriften Nr. 2 123 236, 2 601 780).

So kann man z. B. eine Verbindung der Formel I erhalten durch Umsetzung

a) einer Verbindung der Formel II

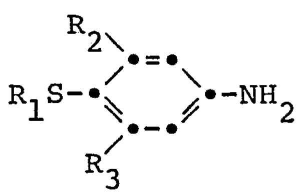

(II)

mit einer Verbindung der Formel III

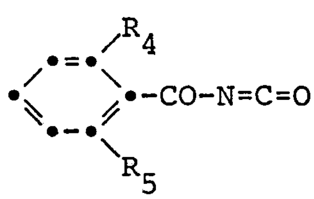

(III)

oder

b) einer Verbindung der Formel IV

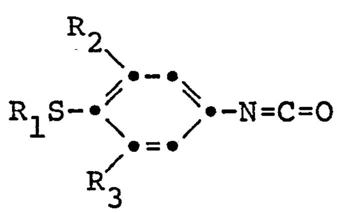

(IV)

gegebenenfalls in Gegenwart einer basischen Substanz mit einer Verbindung der Formel V

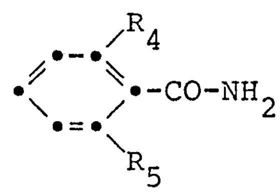

(V)

In den obigen Formeln II, III, IV und V haben die Reste $R_1$, $R_2$, $R_3$, $R_4$, $R_5$ und $R_6$ die unter Formel I vorstehend angegebenen Bedeutungen.

Die erwähnten Verfahren a) und b) können vorzugsweise unter normalem Druck und in Gegenwart eines organischen Lösungs- oder Verdünnungsmittels durchgeführt werden. Als Lösungs- oder Verdünnungsmittel eignen sich z. B. Äther und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid, sowie Ketone, z. B. Aceton, Methyläthylketon, Methylisopropylketon und Methylisobutylketon. Verfahren a) wird im allgemeinen bei einer Temperatur von −10 bis 100°C, vorzugsweise zwischen 15 und 25°C, gegebenenfalls in Gegenwart einer organischen Base, z. B. Triäthylamin, durchgeführt. Die Durchführung von Verfahren b) erfolgt bei einer Temperatur von 0 bis 150°C, vorzugsweise beim Siedepunkt des verwendeten Lösungsmittels, gegebenenfalls in Gegenwart einer organischen Base, wie Pyridin, und/oder unter Zusatz eines Alkali- oder Erdalkalimetalls, vorzugsweise Natrium.

Die Ausgangsstoffe der Formeln II, III, IV und V sind bekannt oder können analog bekannten Verfahren hergestellt werden (vgl. z. B. GB-A-1 141 183 und DE-A-1 193 047).

Es ist bereits bekannt, daß bestimmte substituierte N-Phenyl-N'-benzoylharnstoffe insektizide Eigenschaften besitzen (vgl. DE-A-2 123 236, 2 504 982, 2 531 279 und 2 537 413 sowie BE-A-832 304, 843 906 und 844 066). In US-A-4 089 975 und DE-A-2 601 780 und 2 726 684 werden N-Alkylthiophenyl-N'-benzoylharnstoffe bzw. N-Halogenalkylthiophenyl-N'-benzoylharnstoffe und deren Verwendung als Insektizide beschrieben. Verbindungen ähnlicher Struktur sind auch aus J. Agr. Food Chemistry Vol. 21, (3) 348 (1973) bekannt. Diese bekannten Verbindungen besitzen nur eine verhältnismäßig niedrige insektizide Wirkung.

Überraschenderweise wurde demgegenüber gefunden, daß die erfindungsgemäßen N-Alkenylthiophenyl-N'-benzoylharnstoffe der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Schädlingen, die Pflanzen und Tiere befallen.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Neben ihrer Wirkung gegenüber Fliegen, wie z. B. Musca domestica, und Mückenlarven können Verbindungen der Formel I auch zur Bekämpfung von pflanzenschädigenden Fraßinsekten in Zier- und Nutzpflanzen, insbesondere in Baumwollkulturen (z. B. gegen Spodoptera littoralis und Heliothis virescens), sowie in Obst- und Gemüsekulturen (z. B. gegen Leptinotarsa decemlineata, Laspeyresia pomonella und Epilachna varivestis) eingesetzt werden. Hierbei zeichen sich die Verbindungen der Formel I durch hohe larvizide und vor allem sehr gute ovizide Wirksamkeit aus. Werden Verbindungen der Formel I von adulten Insekten mit dem Futter aufgenommen, so ist in vielen Fällen, insbesondere bei Coleopteren, wie z. B. Anthonomus grandis, eine verminderte Ei-Ablage und/oder reduzierte Schlupfrate festzustellen.

Die Verbindungen der Formel I eignen sich weiterhin zur Bekämpfung von Ektoparasiten an Haus- und Nutztieren, z. B. durch Tier-, Stall- und Weidebehandlung.

Die Wirkung der erfindungsgemäßen Verbindungen bzw. der sie enthaltenden Mittel läßt sich durch Zusatz von anderen Insektiziden und/oder Akariziden wesentlich verbreitern und an gegebene Umstände anpassen.

Als Zusätze kommen z. B. folgende Wirkstoffe in Betracht:

organische Phosphorverbindungen,
Nitrophenole und Derivaten,
Formamidine, Harnstoffe,
Carbamate und
chlorierte Kohlenwasserstoffe.

Mit besonderem Vorteil kann man die Verbindungen der Formel I auch mit Substanzen kombinieren, welche einen pestizid verstärkenden Effekt ausüben. Beispiele solcher Verbindungen sind u. a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithionate.

Die Verbindungen der Formel I können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen, wie z. B. natürlichen oder regenerierten Stoffen, Lösungs-, Dispergier-, Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden. Ferner sind »cattle dips«, d. h. Viehbäder, und »spray races«, d. h. Sprühgänge, in denen wäßrige Zubereitungen verwendet werden, zu erwähnen. Diese Zubereitungsformen sind insbesondere zur Bekämpfung tierparasitärer Schädlinge geeignet.

Die Herstellung erfindungsgemäßer Mittel erfolgt in an sich bekannter Weise durch inniges Vermischen und/oder Vermahlen von Wirkstoffen der Formel I mit den geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen:
Stäubemittel, Streumittel,
Granulate (Umhüllungsgranulate,
Imprägnierungsgranulate und
Homogengranulate);

Flüssige Aufarbeitungsformen:
a) in Wasser dispergierbare Wirkstoffkonzentrate: Spritzpulver (wettable powders), Pasten, Emulsionen;
b) Lösungen:

Der Gehalt an Wirkstoff in den oben beschriebenen Mitteln liegt zwischen 0,1 bis 95%.
Die Wirkstoffe der Formel I können beispielsweise wie folgt formuliert werden:

## Stäubemittel

Zur Herstellung eines a) 5%igen und b) 2%igen Stäubemittels werden die folgenden Stoffe verwendet:

a) 5 Teile Wirkstoff,
95 Teile Talkum;
b) 2 Teile Wirkstoff,
1 Teil hochdisperse Kieselsäure
97 Teile Talkum.

Die Wirkstoffe werden mit den Trägerstoffen vermischt und vermahlen.

## Granulat

Zur Herstellung eines 5%igen Granulates werden die folgenden Stoffe verwendet:

5,00 Teile Wirkstoff,
0,25 Teile epoxydiertes Pflanzenöl,
0,25 Teile Cetylpolyglykoläther,
3,50 Teile Polyäthylenglykol,
91,00 Teile Kaolin (Korngröße 0,3–0,8 mm).

Die Aktivsubstanz wird mit dem epoxydierten Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschließend das Aceton im Vakuum verdampft.

## Spritzpulver

Zur Herstellung eines a) 40%igen, b) und c) 25%igen d) 10%igen Spritzpulvers werden folgende Bestandteile verwendet:

a) 40 Teile Wirkstoff,
5 Teile Ligninsulfonsäure-Natriumsalz,
1 Teil Dibutylnaphthalinsulfonsäure-Natriumsalz,
54 Teile Kieselsäure;

b) 25,0 Teile Wirkstoff,
4,5 Teile Calcium-Ligninsulfonat,
1,9 Teile Champagne-Kreide/Hydroxyäthylcellulose-Gemisch (1 : 1),
1,5 Teile Natrium-dibutyl-naphthalinsulfonat,
19,5 Teile Kieselsäure,
19,5 Teile Champagne-Kreide,
28,1 Teile Kaolin;

c) 25,0 Teile Wirkstoff,
2,5 Teile Isooctylphenoxy-polyoxyäthylen-äthanol,
1,7 Teile Champagne-Kreide/Hydroxyäthylcellulose-Gemisch (1 : 1),
8,3 Teile Natriumaluminiumsilikat,
16,5 Teile Kieselgur,
46,0 Teile Kaolin;

d) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen jeder gewünschten Konzentration verdünnen lassen.

## Emulgierbare Konzentrate

Zur Herstellung eines a) 10%igen b) 25%igen und c) 50%igen emulgierbaren Konzentrates werden folgende Stoffe verwendet:

a) 10,0 Teile Wirkstoff
3,4 Teile epoxydiertes Pflanzenöl,
3,4 Teile eines eines Kombinationsemulgators, bestehend aus Fettalkoholpolyglykoläther und Alkylaralkyl-sulfonat-Calcium-Salz,
40,0 Teile Dimethylformamid,
43,2 Teile Xylol;

b) 25,0 Teile Wirkstoff,
2,5 Teile epoxydiertes Pflanzenöl,
10,0 Teile eines Alkylarylsulfonat/Fettalkoholpolyglykoläther-Gemisches,
5,0 Teile Dimethylformamid,
57,5 Teile Xylol.

c) 50,0 Teile Wirkstoff,
4,2 Teile Tributylphenol-Polyglykoläther,
5,8 Teile Calcium-Dodecylbenzolsulfonat,
20,0 Teile Cyclohexanon,
20,0 Teile Xylol.

Aus solchen Konzentraten können durch Verdünnen mit Wasser Emulsionen jeder gewünschten Konzentration hergestellt werden.

## Sprühmittel

Zur Herstellung eines a) 5%igen und b) 95%igen Sprühmittels werden die folgenden Bestandteile verwendet:

a) 5 Teile Wirkstoff,
1 Teil epoxydiertes Pflanzenöl,
94 Teile Benzin (Siedegrenzen 160 – 190° C);

b) 95 Teile Wirkstoff,
5 Teile epoxydiertes Pflanzenöl

Beschreibung des Herstellungsverfahrens

Zu einer Lösung von 4,95 g 4-Allylthioanilin in 25 ml absolutem Äther werden bei Raumtemperatur und unter Ausschluß von Feuchtigkeit 5,8 g 2,6-Difluorbenzoylisocyanat zugesetzt. Der nach kurzer Zeit ausfallende Niederschlag wird abgesaugt und mit Äther gewaschen. Durch Umkristallisieren aus Toluol erhält man N-(4-Allylthio)-phenyl-N'-2,6-difluorbenzoylharnstoff vom Schmelzpunkt 164–165° C.

Beispiel 1

Analog den vorstehend beschriebenen Arbeitsweisen und der vorstehenden Beschreibung des Herstellungsverfahrens werden die folgenden erfindungsgemäßen Verbindungen der Formel I hergestellt:

| $R_1$ | $R_2$ | $R_3$ | $R_4$ | $R_5$ | Schmelzpunkt [° C] |
|---|---|---|---|---|---|
| $-CH_2-CH=CHCl$ | Cl | Cl | F | F | 156–160*) |
| $-CH_2-CH=CHBr$ | Cl | Cl | F | F | 144,5–146*) |
| $-CH_2-CH=CHCl$ | Cl | Cl | H | F | 164–165*) |

*) Stereoisomerengemisch.

| $R_1$ | $R_2$ | $R_3$ | $R_4$ | $R_5$ | Schmelzpunkt [° C] |
|---|---|---|---|---|---|
| $-CH_2-CH=CHBr$ | Cl | Cl | H | F | 145–149,5*) |

*) Stereoisomerengemisch.

Beispiel 2

Wirkung gegen Musca domestica

Je 50 g frisch zubereitetes CSMA-Nährsubstrat für Maden wurde in Becher eingewogen. Von einer 1gewichtsprozentigen acetonischen Lösung des betreffenden Wirkstoffes wurde eine bestimmte Menge auf das in den Bechern befindliche Nährsubstrat pipettiert. Nach dem Durchmischen des Substrates läßt man das Aceton mindestens 20 Stunden lang verdampfen.

Dann wurden pro Wirkstoff und Konzentration je 25 eintägige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefäßen deponiert.

Die pro Ansatz ausgeschwemmten Puppen wurden gezählt (toxischer Einfluß des Wirkstoffes auf die Madenentwicklung). Dann wurde nach 10 Tagen die Anzahl der aus den Puppen geschlüpften Fliegen bestimmt.

Verbindungen gemäß Beispiel 1 zeigten gute Wirkung im obigen Test.

Beispiel 3

Wirkung gegen Lucilia sericata

Zu 9 ml eines Zuchtmediums wurden bei 50° C 1 ml einer 0,5% Aktivsubstanz enthaltenden wäßrigen Lösung gegeben. Nun wurden ca. 30 frisch geschlüpfte Lucilia-sericata-Larven zum Zuchtmedium gegeben und nach 48 und 96 Stunden die insektizide Wirkung durch Ermittlung der Abtötungsrate festgestellt.

Verbindungen gemäß Beispiel 1 zeigten in diesem Test gute Wirkung gegen Lucilia sericata.

Beispiel 4

Wirkung gegen Aëdes aegypti

Auf die Oberfläche von 150 ml Wasser, das sich in einem Behälter befindet, wurde so viel einer 0,1%igen acetonischen Lösung des Wirkstoffes pipettiert, daß Konzentrationen von je 10, 5 und 1 ppm erhalten wurden. Nach Verdunsten des Acetons wurde der Behälter mit 30—40 2tägigen Aëdes-Larven beschickt. Nach 1, 2 und 5 Tagen wurde die Mortalität bestimmt.

Verbindungen gemäß Beispiel 1 zeigten in diesem Test gute Wirkung gegen Aëdes aegypti.

Beispiel 5

Wirkung gegen Spodoptera littoralis und Heliothis virescens
(Larven; Fraß- und Kontaktwirkung)

Eingetopfte ca. 30 cm hohe Baumwoll- bzw. Soja-Pflanzen wurden mit einer verdünnten, wäßrigen Emulsions-Zubereitung des zu prüfenden Wirkstoffes bis zum Abtropfen besprüht. Nach Antrocknen des Spritzbelages wurden die Baumwoll-Pflanzen mit je 5 Larven im dritten Larvalstadium von Spodoptera und die Soja-Pflanzen mit je 10 Larven im dritten Larvalstadium von Heliothis besetzt. Die Ansätze wurden 5 Tage bei künstlichem Licht, einer Temperatur von etwa 26°C und 50—60% relativer Luftfeuchtigkeit gehalten.

Die Auswertung erfolgte hinsichtlich prozentualer Mortalität, Fraßhemmung, Deformationen und Entwicklungshemmungen im Vergleich zu unbehandelten Kontrollen.

Die Verbindungen gemäß Beispiel 1 zeigten in diesem Test gute Wirkung.

Beispiel 6

Wirkung auf Laspeyresia pomonella (Eier)

Abgelegte Eier von Laspeyresia pomonella, die nicht älter als 24 Stunden waren, wurden auf Filterpapier für 1 Minute in eine acetonisch-wäßrige Lösung enthaltend 400 ppm des zu prüfenden Wirkstoffes eingetaucht. Nach dem Antrocknen der Lösung wurden die Eier in Petrischalen ausgelegt und bei einer Temperatur von 28°C belassen. Nach 6 Tagen wurde der prozentuale Schlupf aus den behandelten Eiern bewertet. Verbindungen gemäß Beispiel 1 zeigten gute Wirkung in obigem Test.

Beispiel 7

Ovizide Wirkung auf Spodoptera littoralis

Auf Filterpapier abgelegte Eier von Spodoptera littoralis wurden aus dem Papier ausgeschnitten und in eine 0,05% gewichtsprozentige Lösung des Wirkstoffes in einem Aceton-Wasser-Gemisch (1 : 1) getaucht. Die so behandelten Eiablagen wurden dann aus diesem Gemisch herausgenommen und bei 21°C und 60% relativer Feuchtigkeit in Kunststoffschalen deponiert.

Nach 3 bis 4 Tagen wurde die Schlupfrate, d. h. die Anzahl der Larven, die sich aus den behandelten Eiern entwickelt hatten, bestimmt.

Verbindungen gemäß Beispiel 1 zeigten gute Wirkung in obigem Test.

Beispiel 8

Wirkung gegen Epilachna varivestis (Larven)

Etwa 15—20 cm hohe Phaseolus-vulgaris-Pflanzen (Buschbohnen) wurden mit einer wäßrigen, den zu prüfenden Wirkstoff enthaltenden Emulsions-Zubereitung besprüht. Nach dem Antrocknen des Sprühbelages wurden pro Pflanze 10 Larven von Epilachna varivestis (Mexikanischer Bohnenkäfer) im 4. larvalen Stadium angesetzt. Über die behandelten Pflanzen wurde ein Plastikzylinder gestülpt, der mit einem Kupfergaze-Deckel abgedeckt war.

Nach 1 und 2 Tagen wurde die akute Wirkung (%-Mortalität) bestimmt. Zur Auswertung hinsichtlich allfälligem Fraß-Schaden (Antifeeding-Effekt), Entwicklungs- und Häutungsstörungen wurden die Versuchstiere während weiterer 3 Tage beobachtet.

Verbindungen gemäß Beispiel 1 zeigten gute Wirkung in obigem Test.

### Beispiel 9

Wirkung gegen Leptinotarsa decemlineata (Larven)

15 cm hohe in Kulturgefäßen befindliche Kartoffelpflanzen, wurden mit einer wäßrigen Emulsions-Zubereitung, enthaltend den zu prüfenden Wirkstoff in einer Konzentration von 500 ppm, gleichmäßig bis zur Tropfnässe mit einer Druckluftspritze besprüht. Nach dem Trocknen der Pflanzen, d. h. nach etwa 1,5 Std., wurde über dieselben ein Plastikzylinder gestülpt, und pro Pflanze wurden je 10 Kartoffelkäferlarven des 3. Stadiums angesetzt. Die Zylinder wurden dann mit einem Kupfergaze-Deckel abgeschlossen und in der Dunkelheit bei 28° C und 60% rel. Luftfeuchtigkeit stehen gelassen.

Nach 1 und 2 Std. sowie 1, 2 und 8 Tagen wurde auf Mortalität der Versuchstiere (Rückenlage) und % — Fraß-Schaden an den Pflanzen geprüft.

Verbindungen gemäß Beispiel 1 zeigten gute Wirkung in obigem Test.

### Beispiel 10

Chemosterilisierende Wirkung auf Anthonomus grandis

Adulte Anthonomus grandis, die nach dem Schlupf nicht älter als 24 Stunden waren, wurden in Gruppen zu jeweils 25 Käfern in Käfige mit Gitterwänden überführt. Die mit den Käfern besetzten Käfige wurden sodann während 5 bis 10 Sekunden in eine acetonische Lösung, enthaltend 1,0 Gew.-% des zu prüfenden Wirkstoffes, eingetaucht.

Nachdem die Käfer wieder trocken waren, wurden sie zur Kopulation und Eiablage in abgedeckte und Futter enthaltende Schalen eingesetzt. Abgelegte Eier wurden zwei- bis dreimal wöchentlich mit fließendem Wasser ausgeschwemmt, gezählt, durch zwei- bis dreistündiges Einlegen in ein wäßriges Desinfektionsmittel (wie z. B. »Actamer B 100«) desinfiziert und dann in Schalen, die eine geeignete Larvaldiät enthielten, deponiert. Nach 7 Tagen wurde untersucht, ob sich aus den deponierten Eiern Larven entwickelt hatten.

Zur Ermittlung der Dauer des Chemosterilans-Effektes der zu prüfenden Wirkstoffe wurde die Eiablage der Käfer während eines Zeitraumes von etwa vier Wochen überprüft. Die Bonitierung erfolgte anhand der Verminderung der Anzahl abgelegter Eier und geschlüpfter Larven im Vergleich zu unbehandelten Kontrollen.

Verbindungen gemäß Beispiel 1 zeigten gute Wirkung in obigem Test.

**Patentansprüche**

1. Verbindung der Formel I

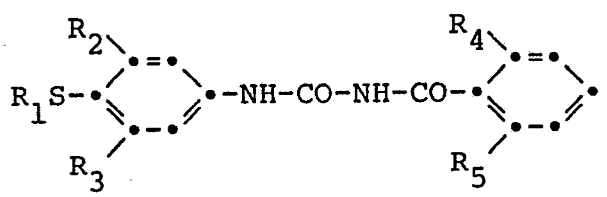

(I)

worin
$R_1$ den Rest $-CH_2-CH=CH-R_6$
$R_2$ und $R_3$ Chlor;
$R_4$ Wasserstoff oder Fluor;
$R_5$ Fluor und
$R_6$ Chlor oder Brom
bedeuten.

2. Verbindung gemäß Anspruch 1 der Formel

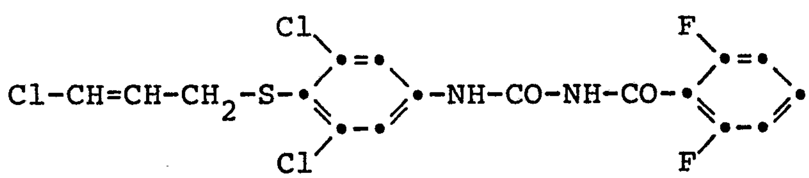

3. Verbindung gemäß Anspruch 1 der Formel

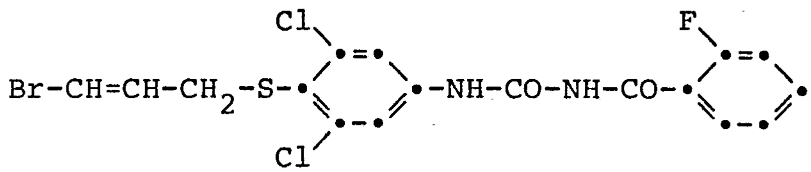

4. Verfahren zur Herstellung einer Verbindung gemäß den Ansprüchen 1 bis 3, dadurch gekennzeichnet, daß man

a) eine Verbindung der Formel II

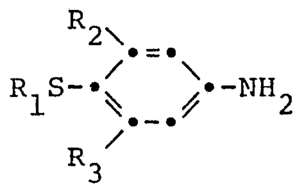

(II)

mit einer Verbindung der Formel III

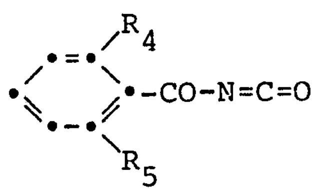

(III)

oder

b) eine Verbindung der Formel IV

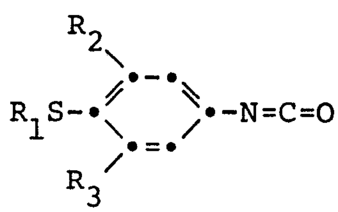

(IV)

mit einer Verbindung der Formel V

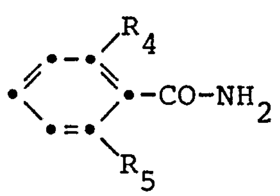

(V)

umsetzt, wobei in den Formeln II bis V die Reste $R_1$, $R_2$, $R_3$, $R_4$ und $R_5$ die im Anspruch 1 angegebenen Bedeutungen haben.

5. Schädlingsbekämpfungsmittel, welches als aktive Komponente eine Verbindung gemäß den Ansprüchen 1 bis 3 zusammen mit geeigneten Trägern und/oder anderen Zuschlagstoffen enthält.

6. Verwendung einer Verbindung gemäß den Ansprüchen 1 bis 3 zur Bekämpfung von Schädlingen, vorzugsweise von Insekten.

7. Verwendung gemäß Anspruch 6 als Ovizide zur Bekämpfung von Insekten, vorzugsweise pflanzenschädigenden Insekten.

**Claims**

1. A compound of the formula I

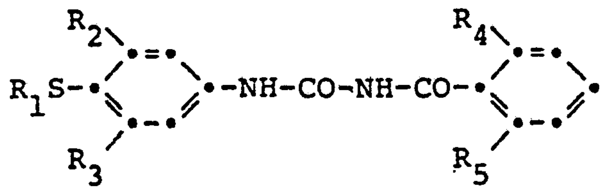

(I)

wherein
$R_1$ is the radical $-CH_2-CH=CH-R_6$,
$R_2$ and $R_3$ are chlorine,
$R_4$ is hydrogen or fluorine,
$R_5$ is fluorine, and
$R_6$ is chlorine or bromine.

2. A compound according to Claim 1 of the formula

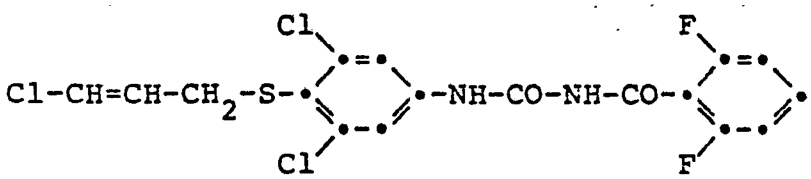

3. A compound according to Claim 1 of the formula

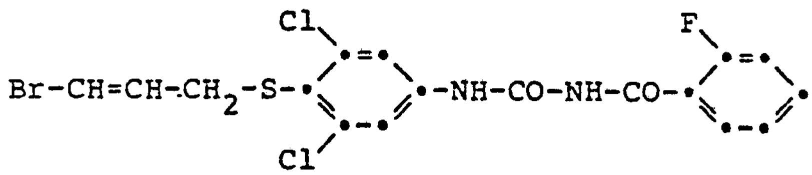

4. A process for producing a compound according to Claims 1 to 3, which process comprises reacting

a) a compound of the formula II

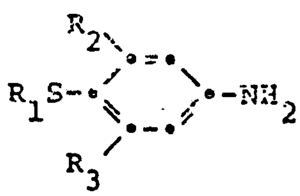

(II)

with a compound of the formula III

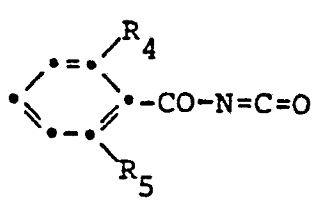

(III)

or
b) a compound of the formula IV

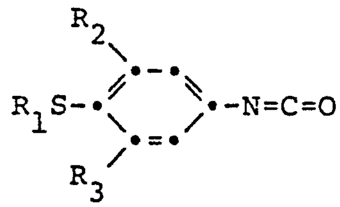

(IV)

with a compound of the formula V

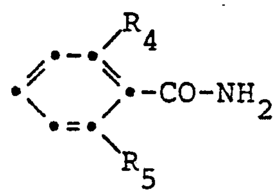

(V)

the radicals $R_1$, $R_2$, $R_3$, $R_4$ and $R_5$ in the formulae II to V having the meanings defined in Claim 1.

5. A pesticidal composition which contains as active ingredient a compound according to Claims 1 to 3, together with suitable carriers and/or other additives.

6. Use of a compound according to Claims 1 to 3 for combating pests, particularly insect pests.

7. Use according to Claim 6 as ovicides for combating insect pests, particularly insects which damage plants.

**Revendications**

1. Composé de formule I

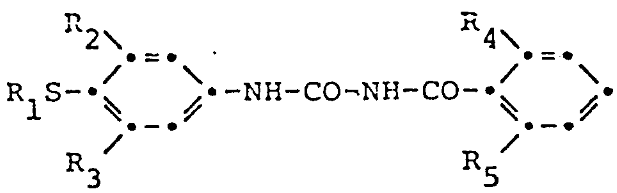

(I)

où
$R_1$ représente le radical $-CH_2-CH=CH-R_6$
$R_2$ et $R_3$ représentent un chlore,
$R_4$ représente un hydrogène ou un fluor,
$R_5$ représente un fluor, et
$R_6$ représente un chlore ou un brome.

2. Composé selon la revendication 1 de formule

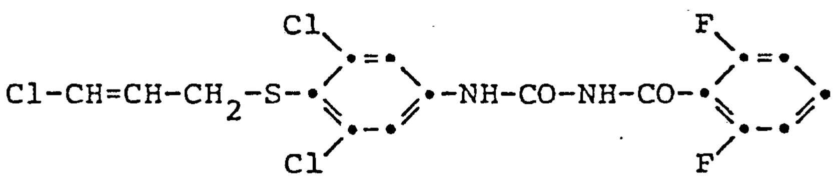

3. Composé selon la revendication 1 de formule

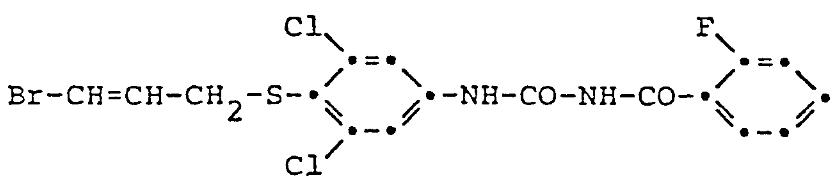

4. Procédé de préparation d'un composé selon les revendications 1 à 3, caractérisé en ce que

a) on fait réagir un composé de formule II

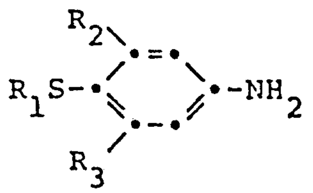

(II)

avec un composé de formule III

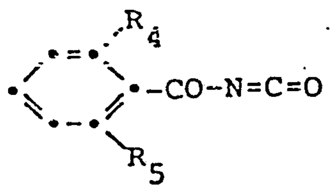

(III)

ou

b) on fait réagir un composé de formule IV

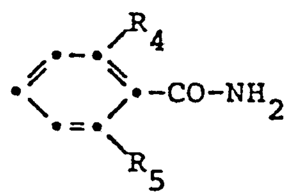

(V)

avec un composé de formule V

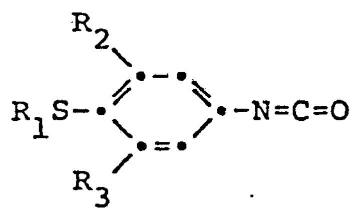

(IV)

Où dans les formules II à V les radicaux $R_1$, $R_2$, $R_3$, $R_4$ et $R_5$ ont les significations données dans la revendication 1.

5. Agent de lutte antiparasitaire contenant comme composant actif un composé selon les revendications 1 à 3 avec des supports et/ou autres additifs appropriés.

6. Application d'un composé selon les revendications 1 à 3 pour lutter contre les parasites, de préférence les insectes.

7. Application selon la revendication 6 comme ovicide pour lutter contre les insectes, de préférence les insectes causant des dommages aux plantes.